Europäisches Patentamt

European Patent Office

Office européen des brevets

(11) Publication number: **0 129 201**

A2

# EUROPEAN PATENT APPLICATION

(21) Application number: 84106759.8

(22) Date of filing: 13.06.84

(51) Int. Cl.³: **A 61 K 7/18**

(30) Priority: 16.06.83 GB 8316382

(43) Date of publication of application:
27.12.84 Bulletin 84/52

(84) Designated Contracting States:
AT BE CH DE FR GB IT LI NL SE

(71) Applicant: BEECHAM GROUP PLC
Beecham House Great West Road
Brentford Middlesex TW8 9BD(GB)

(72) Inventor: Duke, Susan Ann
6 Clifton Close Addlestone
Weybridge Surrey, KT15 2EX(GB)

(72) Inventor: Pearl, Adrian Clive
1 Fairview Drive
Watford Hertfordshire(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) Oral hygiene compositions.

(57) A dentifrice composition comprising
(a) a dentally acceptable abrasive selected from calcium carbonate, dicalcium phosphate, an insoluble silicate, calcium bicarbonate, insoluble sodium metaphosphate, alumina and a silica; and
(b) an aqueous solution of alkali metal fluoride, alkali metal monofluorophosphate and orthophosphate anions, the latter having a concentration of at least 0.5mM in the aqueous solution, and the solution having a pH of from 7 to 11.

EP 0 129 201 A2

C207

ORAL HYGIENE COMPOSITIONS

The present invention relates to oral hygiene
compositions, and in particular to dentifices having
improved anticaries effect.

A variety of anticaries agents have hitherto been used
in dentifrices, including alkali metal fluorides and
alkali metal monofluorophosphates. It has now been
discovered that enhanced anticaries effect can be
achieved when a combination of alkali metal fluoride,
alkali metal monofluorophosphate and a dissolved
orthophosphate are used within a certain pH range.

According to the present invention there is provided a
dentifrice composition comprising:

(a) a dentally acceptable abrasive selected from
calcium carbonate, dicalcium phosphate, an insoluble
silicate, calcium bicarbonate, insoluble sodium
metaphosphate, alumina and a silica; and

(b) an aqueous solution of alkali metal fluoride,
alkali metal monofluorophosphate and orthophosphate
anions, the latter having a concentration of at least
0.5mM in the aqueous solution, and the solution having
a pH of from 7 to 11.

The dentally acceptable abrasive may comprise one or
more of the listed materials, each of which is well
known in the art for its abrasive use in dentifrices.
The amount of abrasive will be within conventional
limits, and will normally vary from about 15% to 50% by
weight of the composition.

The preferrred alkali metal fluoride is sodium
fluoride, and the preferred alkali metal
monofluorophosphate is sodium monofluorophosphate.
The composition of the invention preferably comprises
from 100 ppm to 2500 ppm of fluoride ions in total.
Preferably, from 10 to 90%, more preferably 40 to 80%,
of the fluoride is provided by the alkali metal
fluoride and the remainder is provided by the alkali
metal monofluorophosphate.

Preferred sources of orthophosphate anions are
trisodium orthophosphate, disodium hydrogen
orthophosophate, tripotassium orthophosphate or
dipotassium hydrogen orthophosphate, particularly
trisodium or disodium hydrogen orthophosphate.  Other
orthophosphate salts may be used in place of these or
in combination with these but if less basic
orthophosphates are employed it may be necessary to
buffer the solution to achieve the desired pH.

The quantity of orthophosphate salt required to give
the necessary orthophosphate ion concentration can
routinely be calculated from the molecular weight of a
particular salt.

Preferably the solution comprises at least 1m$\underline{M}$
dissolved orthophosphate anions, particularly when the
solution has a pH from 7 to 8.

- 3 -

Preferably the aqueous solution has a pH from 7.5 to 10.5 more preferably pH 8 or above, especially pH 8.5 to 10.5.

The compositions of the invention may optionally contain other agents known to enhance the anticaries effect of fluoride and monofluorphosphate, such as calcium glycerophosphate, this being incorporated in a weight ratio of up to 1:3, preferably 1:20 to 1:3, to the weight of fluoride and monofluorophosphate salts. If the composition of the invention contains any ingredient which releases cations capable of forming a stable, insoluble fluoride salt, then the composition also preferably includes dissolved carbonate, bicarbonate or other dissolved anions capable of forming a more stable, insoluble salt with the cations. For instance a composition containing calcium carbonate abrasive would also contain a soluble carbonate or bicarbonate such as sodium carbonate or bicarbonate in the aqueous solution.

The composition of the invention may be presented in conventional toothpaste, dental cream and dental powder formulations. In the latter case the aqueous solution is formed during the slurrying operation prior to or during application of the powder to the teeth.

Compositions according to the invention have a two-fold effect on caries - the fluoride agents cause formation of fluorohydroxy apatite in the enamel and thereby make the teeeth more resistant to caries attack, and they reduce plaque growth and increase plaque pH thereby reducing acid formation and hence reducing the severity of attack on the tooth enamel. Both these effects are enhanced by the orthophosphate as shown in the data section below.

- 4 -

Compositions according to the present invention may be produced by admixture of the various ingredients and may contain the usual optional accessory ingredients such as dyes, sweetening agents, for example soluble saccharin, flavouring oils, for example oils of spearmint, wintergreen or peppermint, chloroform, colouring or whitening agents for example titanium dioxide, preservatives for example sodium benzoate, emulsifying agents, silicones, alcohol, menthol chlorophyll compounds for example sodium copper chlorophyllin, antibacterial agents, for example chlorhexidine, anti-plaque agents, anti-calculus agents, agents for sensitive dentine for example strontium salts or formaldehyde, and agents which enhance the anticaries activity of fluorides (e.g. calcium glycerophosphate).

The composition of the invention may, accordingly, be prepared by admixing alkali metal fluoride, alkali metal monofluorophosphate and orthophosphate anions in optionally buffered aqueous medium, in relative amounts to produce a pH of from 7 to 11 and combining the resultant aqueous solution with a dentally acceptable abrasive.

The invention is illustrated by the following examples.

- 5 -

Example 1

| PASTE IN SILICA BASE | % w/w |
|---|---|
| Zeodent 113 | 18.00 |
| Sorbitol | 50.00 |
| Syloblanc 34 | 3.50 |
| Glycerin | 4.50 |
| Carboxymethyl cellulose CMC 7MF | 1.00 |
| Flavour C 180 | 0.60 |
| 15% Saccharin solution | 1.20 |
| Sodium monofluorophosphate | 0.38 |
| Sodium fluoride | 0.11 |
| Sodium dihydrogen phosphate ($NaH_2PO_4$ $2H_2O$) | 0.23 |
| Disodium hydrogen phosphate ($Na_2HPO_4$ $12H_2O$) | 0.54 |
| Nipagin Sodium | 0.15 |
| 30% Empicol | 4.00 |
| Calcium glycerophosphate | 0.13 |
| Water | 14.01 |
| Natrosol 250H | 1.50 |
| 0.1% FD ε C Blue No 1 Dye | 0.15 |
| TOTAL | 100.00 |

Zeodent is a trade mark of the Huber Corporation
Syloblanc is a trade mark of W.R. Grace and Co.
Empicol is a trade mark for sodium lauryl sulphate
Natrosol is a trade mark of Hercules Ltd.

- 6 -

Example 2

| PASTE IN CALCIUM CARBONATE BASE | % w/w |
|---|---|
| 70% Sorbitol | 30.00 |
| 15% Saccharin | 2.33 |
| Carboxymethyl cellulose CMC 7MF | 1.20 |
| Natrosol 250H | 0.10 |
| Calcium Silicate | 0.20 |
| Polyvinyl pyrrolidone | 0.10 |
| Calcium glycerophosphate | 0.13 |
| Sodium monofluorophosphate | 0.38 |
| AC 1048P (flavour) | 1.15 |
| Omya D2 | 35.00 |
| Sipernat 225 | 3.50 |
| Titanium dioxide | 0.50 |
| Empicol 0193 | 1.88 |
| Sodium fluoride | 0.11 |
| Sodium carbonate | 0.50 |
| Sodium hydrogen carbonate | 1.50 |
| Sodium dihydrogen phosphate ($NaH_2PO_4.2H_2O$) | 0.50 |
| Disodium hydrogen phosphate ($Na_2HPO_4.12H_2O$) | 0.50 |
| Water to 100% | |

Omya is a trade mark of Prüss Staufer

Sipernat is a trade mark of Degussa Ltd

- 7 -

Test Data

1.  Fluoride uptake:

Fluoride uptake by hydroxyapatite from solutions
containing sodium fluoride or sodium fluoride and
disodium hydrogen phosphate was measured by the method
of Duke and Forward Caries Res. 12, 12-20, (1978).

Results are presented - Table 1.

Table 1

| NaF (mM) | Na$_2$HP04 (mM) | FLUORIDE UPTAKE | |
| --- | --- | --- | --- |
| | | Total F | FHA |
| 5.26 | – | 320 | 100 |
| 5.26 | 0.526 | 317 | 114 |
| 5.26 | 1.05 | 323 | 146 |
| 5.26 | 5.26 | 316 | 163 |

2.  Effect on the pH of dental plaque

20 subjects were randomly allocated to a treatment and
were allowed to use a test toothpaste for 1 week
ad.lib. They then refrained from all oral hygiene
measures for 24 hours. On the test day each subject
refrained from eating or drinking for 1 hour prior to
and during the test period. Each subject rinsed his mouth with 10 ml of a 3:1 slurry in water of the test toothpaste. 30 minutes later plaque samples were collected from the molars and premolars and the mouth was then rinsed with 10ml of 10% w/v aqueous sucrose.

Ten minutes later plaque samples were again taken from the molars and premolars.

Each plaque sample, immediately following sampling, was suspended in 50ml physiological saline and the pH was measured using a microcombination electrode.

The whole procedure was repeated on two further occasions. The subjects then began the alternative treatment and the test repeated.

The results are presented in Table 2.

Measurements of plaque growth were also made by the method of Stean and Forward, Community Dent. Oral Epidemiol. 1980, 8, 420 - 423. The results are also presented in Table 2.

## Table 2

| Treatment | plaque pH | plaque growth |
|---|---|---|
| A Toothpaste containing 26mM NaF | | |
| 26mM $NaPO_2F$ | $6.19 \pm 0.21$ | 7.1 |
| | | |
| B Toothpaste containing 26mM NaF | | |
| 26mM $NaPO_2F$ | $6.33 \pm 0.22$ | 5.0 |
| 15mM $Na_2HPO_4$ | | |
| 15mM $NaH_2PO_4$ | | |

Statistical significance

| plaque pH | B > A $p < 0.001$ |
| plaque growth | B > A $p < 0.05$ |

- 1 -

Claims

1) A dentifrice composition comprising

(a) a dentally acceptable abrasive selected from calcium carbonate, dicalcium phosphate, an insoluble silicate, calcium bicarbonate, insoluble sodium metaphosphate, alumina and a silica; and

(b) an aqueous solution of alkali metal fluoride, alkali metal monofluorophosphate and orthophosphate anions, the latter having a concentration of at least 0.5mM in the aqueous solution, and the solution having a pH of from 7 to 11.

2) A dentifrice according to claim 1 containing from 100 ppm to 2500 ppm of fluoride ions in total.

3) A dentifrice according to claim 1 or 2, in which from 10% to 90% of the fluoride is provided by an alkali metal fluoride, and the remainder is provided by an alkali metal monofluorophosphate.

4) A dentifrice according to any one of claims 1 to 3, in which orthophosphate anions are provided by trisodium orthophosphate, disodium hydrogen orthophosphate, tripotassium orthophosphate or dipotassium hydrogen orthophosphate.

- 2 -

5) A dentifrice according to any one of claim 1 to 4, in which the aqueous solution comprises at least 1mM dissolved orthophosphate anions.

6) A dentifrice according to any one of claims 1 to 5, in which the aqueous solution has a pH of from 8.5 to 10.5.

7) A dentifrice according to any one of claims 1 to 6, comprising from 15 to 50% by weight of dentally acceptable abrasive.

8) A process for preparing a dentifrice according to any one of claims 1 to 7, which comprises admixing alkali metal fluoride, alkali metal monofluorophosphate and orthophosphate anions in optionally buffered aqueous medium, in relative amounts to produce a pH of from 7 to 11, and combining the resultant aqueous solution with a dentally acceptable abrasive.